(19) Europäisches Patentamt
European Patent Office
Office européen des brevets

(11) EP 1 858 982 B1

# (12) EUROPEAN PATENT SPECIFICATION

(45) Date of publication and mention of the grant of the patent:
**27.08.2008 Bulletin 2008/35**

(21) Application number: **06738618.5**

(22) Date of filing: **17.03.2006**

(51) Int Cl.:
*C08L 83/04* (2006.01) *C08K 5/42* (2006.01)
*C08K 5/12* (2006.01) *C08J 3/03* (2006.01)

(86) International application number:
**PCT/US2006/009580**

(87) International publication number:
**WO 2006/102010 (28.09.2006 Gazette 2006/39)**

(54) **SILICONE EMULSION, METHOD OF PREPARING SAME, AND COSMETIC INGREDIENT**

SILIKONMEMULSION, VERFAHREN ZU IHRER HERSTELLUNG UND KOSMETISCHER BESTANDTEIL

EMULSION DE SILICONE, METHODE DE PREPARATION DE LADITE EMULSION, ET INGREDIENT COSMETIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.03.2005 US 662731 P**

(43) Date of publication of application:
**28.11.2007 Bulletin 2007/48**

(73) Proprietor: **Dow Corning Corporation**
**Midland MI 48686-0994 (US)**

(72) Inventor: **JOFFRE, Eric, Jude**
**Midland, Michigan 48640 (US)**

(74) Representative: **Gillard, Richard Edward et al**
**Elkington and Fife LLP**
**Prospect House**
**8 Pembroke Road**
**Sevenoaks**
**Kent TN13 1XR (GB)**

(56) References cited:
**DE-A1- 19 834 814**
**US-A1- 2004 138 373**
**US-A1- 2004 147 647**

- **"Detergent compsn. imparting good smoothness to hair and skin - contg. alkylol alkyl taurine salt type anionic surfactant and silicone derivs" DERWENT, 20 October 1992 (1992-10-20), XP002208331**
- **PATENT ABSTRACTS OF JAPAN vol. 1998, no. 09, 31 July 1998 (1998-07-31) & JP 10 110161 A (SHIN NIPPON KUCHO KK; TOSHIBA SILICONE CO LTD), 28 April 1998 (1998-04-28)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 858 982 B1

## Description

CROSS-REFERENCE TO RELATED APPLICATIONS

This application claims the benefit of U.S. Provisional Patent Application No. 60/662731 filed 17 March 2005 under 35 U.S.C. §119 (e). U.S. Provisional Patent Application No. 60/662731 is hereby incorporated by reference.

FIELD OF THE INVENTION

The present invention relates to a silicone emulsion and more particularly to a silicone emulsion comprising a polyorganosiloxane, a cholic acid derivative, and water, wherein the polyorganosiloxane contains not greater than 10% (w/w) of cyclic organosiloxane tetramer. The present invention also relates to a method of preparing the silicone emulsion and to a cosmetic ingredient containing the emulsion.

BACKGROUND OF THE INVENTION

Silicone emulsions containing various surfactants are generally prepared by suspension polymerization or emulsion polymerization. However, such emulsions typically contain appreciable concentrations of volatile cyclic organosiloxane oligomers, including cyclic organosiloxane tetramers. Moreover, the concentration of the organosiloxane oligomers typically increases with increasing molecular weight of the polyorganosiloxane.

When a silicone emulsion is used for the preparation of cosmetic products, it may be necessary to limit the amount of cyclic organosiloxane oligomers in the emulsion. However, selective removal of residual cyclic organosiloxanes can be difficult and costly. Moreover, methods of removing the cyclic organosiloxane oligomers, for example, pervaporation, may cause degradation of the emulsion.

Therefore, there is a need for a silicone emulsion containing a high molecular weight polyorganosiloxane having a low content of cyclic organosiloxanes.

SUMMARY OF THE INVENTION

The present invention is directed to a silicone emulsion, comprising:

(A) a polyorganosiloxane containing not greater than 10% (w/w) of cyclic organosiloxane tetramer;
(B) a surfactant having the formula:

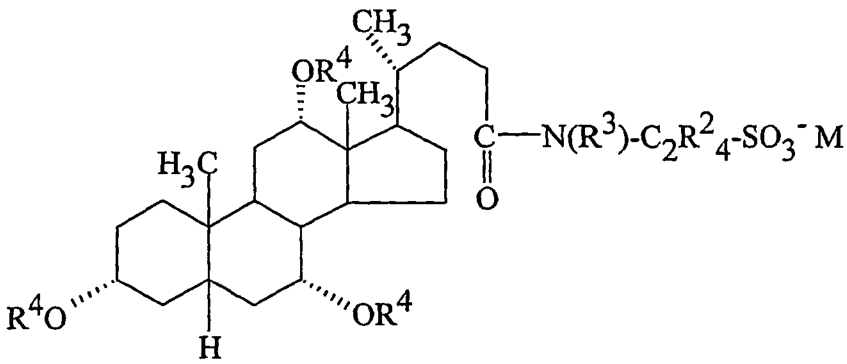

wherein each $R^2$ is independently -H or -F, $R^3$ is -H, hydrocarbyl, or substituted hydrocarbyl, each $R^4$ is independently $R^3$ or $-(CH_2CH_2O)_mR^3$, wherein m is from 1 to 20, and M is a metal ion or an ammonium ion; and
(C) water.

The present invention is also directed to a method of preparing a silicone emulsion, the method comprising:

(i) emulsifying a mixture comprising (A') an organosiloxane having the formula $HO(R^1{}_2SiO)_nH$, where each $R^1$ is independently hydrocarbyl or substituted hydrocarbyl, and n has a value such that the organosiloxane has a weight-average molecular weight of from 92 to 100,000; (B') a surfactant having the formula:

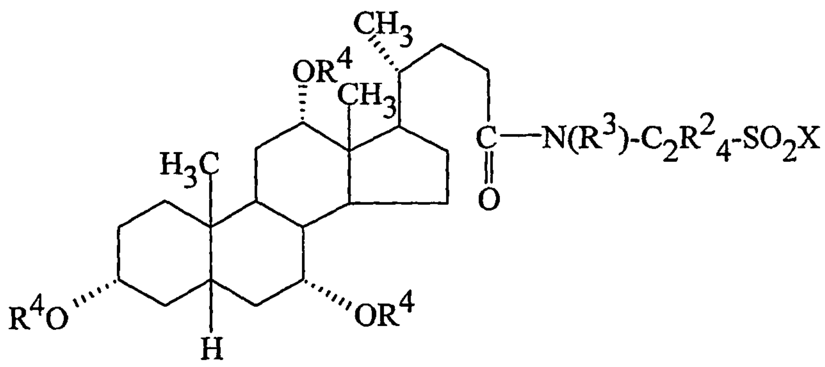

wherein each $R^2$ is independently -H or -F, $R^3$ is -H, hydrocarbyl, or substituted hydrocarbyl, each $R^4$ is independently $R^3$ or $-(CH_2CH_2O)_mR^3$, wherein m is from 1 to 20, and X is -OH or $-O^-$ M, wherein M is a metal ion or an ammonium ion, and (C) water;

(ii) polymerizing the organosiloxane of the emulsified mixture in the presence of an acid catalyst to produce a polyorganosiloxane having a weight-average molecular weight of at least 2 times the weight-average molecular weight of the organosiloxane; and

(iii) neutralizing the acid catalyst.

The present invention is further directed to a cosmetic ingredient comprising the aforementioned silicone emulsion.

The silicone emulsion of the present invention has a very low concentration of cyclic organosiloxane oligomers and high stability. In particular, the silicone emulsion comprises a relatively high molecular weight polyorganosiloxane that contains not greater than 10% (w/w) of cyclic organosiloxane tetramer.

The method of preparing the silicone emulsion utilizes readily available starting materials and conventional equipment. Moreover, the method can be performed in a minimum number of steps, and is scaleable to a manufacturing process. Importantly, the method produces a silicone emulsion containing a very low content of cyclic organosiloxane oligomer.

The silicone emulsion of the present invention is useful as an ingredient in a wide range of consumer products, including paints, coatings, and personal care products. In particular, the silicone emulsion can be used as an ingredient in cosmetic compositions, such as skin creams, foundation, eye shadow, body wash, shampoo, hair rinse, and hair conditioner.

BRIEF DESCRIPTION OF THE DRAWINGS

Figure 1 shows a plot of $\%D_4$, based on total weight of siloxanes, versus weight-average molecular weight of the polyorganosiloxane, for silicone emulsions prepared using three different surfactants, sodium dodecyl sulfate, sodium methyl cocoyl taurate, and sodium taurocholate, at 25 °C.

DETAILED DESCRIPTION OF THE INVENTION

As used herein, the term "cyclic organosiloxane oligomers" refers to organocyclosiloxanes containing from four to twelve silicon atoms. Also, the term "cyclic organosiloxane tetramer" refers to at least one organocyclosiloxane containing four silicon atoms. Furthermore, as used in reference to the present invention, the term "silicone emulsion" refers to a composition containing a colloidal suspension of droplets or particles of a polyorganosiloxane in an aqueous continuous phase, and a surfactant (cholic acid derivative).

A silicone emulsion according to the present invention comprises:

(A) a polyorganosiloxane containing not greater than 10% (w/w) of cyclic organosiloxane tetramer;

(B) a surfactant having the formula:

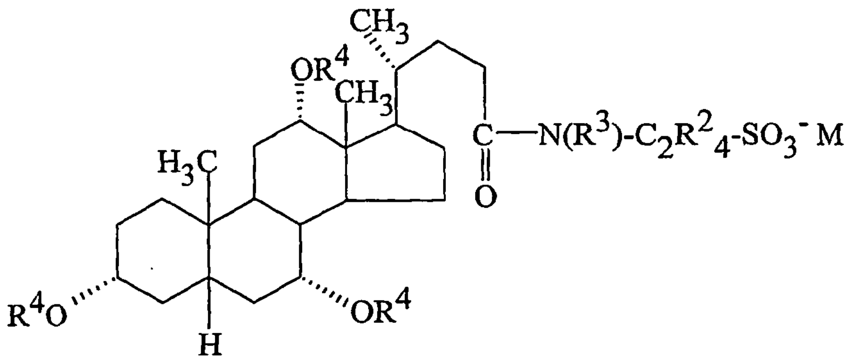

wherein each $R^2$ is independently -H or -F, $R^3$ is -H, hydrocarbyl, or substituted hydrocarbyl, each $R^4$ is independently $R^3$ or $-CH_2CH_2O)_mR^3$, wherein m is from 1 to 20, and M is a metal ion or an ammonium ion; and
(C) water.

Component (A) is at least one polyorganosiloxane. The polyorganosiloxane can have a linear, branched, or resinous structure. Also, the polyorganosiloxane can be a homopolymer or a copolymer. The silicon-bonded organic groups in the polyorganosiloxane are typically hydrocarbyl or substituted hydrocarbyl. In addition to these groups, the polyorganosiloxane can contain silicon-bonded hydroxy groups or silicon-bonded alkyloxy groups.
The hydrocarbyl and substituted hydrocarbyl groups typically have from 1 to 20 carbon atoms, alternatively from 1 to 10 carbon atoms, alternatively from 1 to 6 carbon atoms.
Examples of hydrocarbyl groups include, but are not limited to, alkyl, such as methyl, ethyl, propyl, 1-methylethyl, butyl, 1-methylpropyl, 2-methylpropyl, 1,1-dimethylethyl, pentyl, 1-methylbutyl, 1-ethylpropyl, 2-methylbutyl, 3-methylbutyl, 1,2-dimethylpropyl, 2,2-dimethylpropyl, hexyl, heptyl, octyl, nonyl, decyl, undecyl, dodecyl, tridecyl, tetradecyl, pentadecyl, hexadecyl, heptadecyl, and octadecyl; cycloalkyl, such as cyclopentyl, cyclohexyl, and methylcyclohexyl; aryl, such as phenyl and naphthyl; alkaryl, such as tolyl and xylyl; aralkyl, such as benzyl and phenethyl; alkenyl, such as vinyl, allyl, and propenyl; arylalkenyl, such as styryl and cinnamyl; and alkynyl, such as ethynyl and propynyl.
The substituted hydrocarbyl groups can contain one or more of the same or different substituents, provided the substituent does not adversely affect the stability of the silicone emulsion. Examples of substituents include, but are not limited to, halo, epoxy, carboxy, amino, acryloyl, methacryloyl, and mercapto.
The alkyloxy groups typically have from 1 to 8 carbon atoms, alternatively from 1 to 4 carbon atoms. Examples of alkyloxy groups include, but are not limited to, methoxy, ethoxy, propoxy, butoxy, and pentyloxy.
The polyorganosiloxane typically has a weight-average molecular weight of from 10,000 to 10,000,000, alternatively from 50,000 to 5,000,000, alternatively from 100,000 to 1,000,000, as determined by gel permeation chromatography employing a refractive index detector and polystyrene standards.
Component (A) typically contains not greater than 10% (w/w), alternatively not greater than 5% (w/w), alternatively not greater than 3.5% (w/w), alternatively not greater than 3% (w/w), alternatively not greater than 2.5% (w/w), alternatively not greater than 2% (w/w), alternatively not greater than 1.5% (w/w), of cyclic organosiloxane tetramer, based on the total weight of component (A). The organosiloxane can be a single cyclic organosiloxane tetramer or a mixture of two or more different cyclic organosiloxane tetramers. Examples of organosiloxane tetramers include octamethylcyclotetrasiloxane and octaethylcyclotetra-siloxane. The concentration of organosiloxane tetramer in component (A) can be determined using gel permeation chromatography as described in the Examples section below.
Examples of polyorganosiloxanes include, but are not limited to, the following siloxanes:

α,ω-dihydroxypolydimethylsiloxane,
α-hydroxy-ω-trimethylsiloxypolydimethylsiloxane,
α,ω-dimethoxypolydimethylsiloxane,
α-methoxy-ω-trimethylsiloxypolydimethylsiloxane,
α,ω-diethoxypolydimethylsiloxane,
α-ethoxy-ω-trimethylsiloxypolydimethylsiloxane, and
α,ω-di(trimethylsiloxy)polydimethylsiloxane.

Component (A) can be a single polyorganosiloxane or a mixture comprising two or more different polyorganosilxoxanes, each as described and exemplified above.
Furthermore, the polyorganosiloxane can be prepared as described below in the method of preparing the silicone

emulsion.

Component (B) is at least one surfactant having the formula:

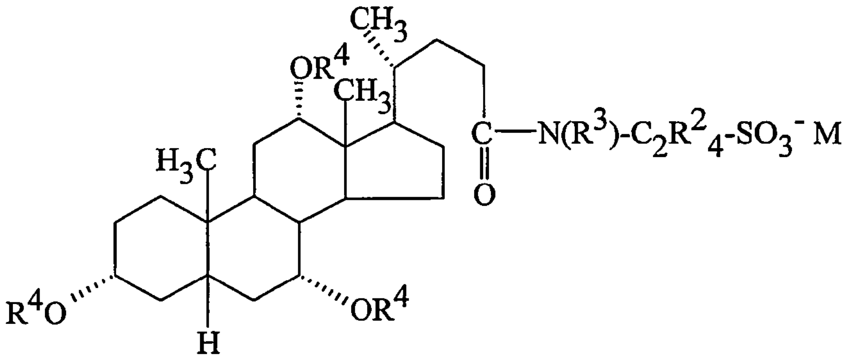

wherein each $R^2$ is independently -H or -F, $R^3$ is -H, hydrocarbyl, or substituted hydrocarbyl, each $R^4$ is independently $R^3$ or $-(CH_2CH_2O)_mR^3$, wherein m is from 1 to 20, and M is a metal ion or an ammonium ion.

The hydrocarbyl and substituted hydrocarbyl groups represented by $R^3$ are as described and exemplified above for the polyorganosiloxane of component (A).

The group $R^4$ can have the formula $-(CH_2CH_2O)_mR^3$, wherein m has a value of from 1 to 20 and $R^3$ is as described and exemplified above. Alternatively m can have a value of from 1 to 15, or from 5 to 15. Examples of the preceding groups include, but are not limited to, groups having the following formulae: $-CH_2CH_2OCH_3$, $-(CH_2CH_2O)_5CH_3$,$-(CH_2CH_2O)_{10}CH_3$, and $-(CH_2CH_2O)_{15}CH_3$.

In the above formula of the surfactant, M is a metal ion or an ammonium ion. Examples of ions represented by M include, but are not limited to, alkali metal ions, such as sodium ion and potassium ion; alkaline earth metal ions, such as magnesium ion; and ammonium ions, such as ammonium ($NH_4^+$) and tris(2-hydroxyethyl)ammonium.

Examples of surfactants suitable for use as component (B) include, but are not limited to, salts of taurocholic acid, such as taurocholic acid sodium salt, taurocholic acid potassium salt, taurocholic acid lithium salt, taurocholic acid magnesium salt, and taurocholic acid triethanolamime salt.

Component (B) can be a single surfactant or a mixture comprising two or more different surfactants, each as described and exemplified above.

The concentration of component (B) is typically from 0.1 to 100 parts by weight, alternatively from 0.1 to 50 parts by weight, alternatively from 0.5 to 10 parts by weight, alternatively from 0.5 to 5 parts by weight, per 100 parts by weight of component (A).

Methods of preparing cholic acid derivatives, including ethers and amides, suitable for use as component (B) are well known in the art.

Component (C) is water, which is the continuous phase of the silicone emulsion. The concentration of water in the silicone emulsion is typically from 30 to 1000 parts by weight, alternatively from 40 to 400 parts by weight, alternatively from 50 to 250 parts by weight, per 100 parts by weight of component (A).

The silicone emulsion can further comprise additional ingredients, provided the ingredient does not adversely affect the stability of the emulsion. Examples of additional ingredients include, but are not limited to, anionic surface active agents such as sodium polyoxyethylene lauryl ether acetate, disodium polyoxyethylene lauryl sulfosuccinate, sodium polyoxyethylene lauryl ether sulfate, sodium α-olefinsulfonate, triethanolamine salt of dodecylbenzenesulfonic acid, and sodium polyoxyethylene lauryl ether phosphate; nonionic surface active agents such as glycerin monostearate, sorbitan monopalmitate, polyoxyethylene cetyl ether, polyoxyethylene lauryl ether, polyoxyethylene stearyl ether, polyoxyethylene stearate, polyoxyethylene sorbitan monolaurate, coconut fatty acid diethanolamide, polyoxyethylene oxypropylene glycol, and modified silicone oil containing polyoxyethylene groups; antiseptics and bactericides; pH-adjusting agents; mildew-proofing agents; and rust preventives.

A method of preparing a silicone emulsion according to the present invention comprises:

(i) emulsifying a mixture comprising (A') an organosiloxane having the formula $HO(R^1{}_2SiO)_nH$, where each $R^1$ is independently hydrocarbyl or substituted hydrocarbyl, and n has a value such that the organosiloxane has a weight-average molecular weight of from 92 100,000; (B') a surfactant having the formula:

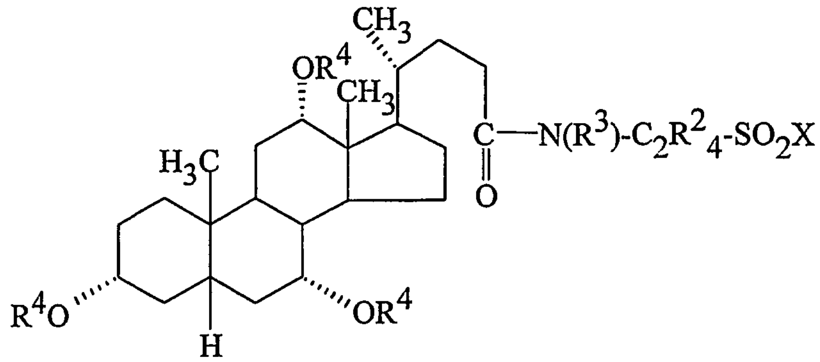

wherein each $R^2$ is independently -H or -F, $R^3$ is -H, hydrocarbyl, or substituted hydrocarbyl, each $R^4$ is independently $R^3$ or $-(CH_2CH_2O)_mR^3$, wherein m is from 1 to 20, and X is -OH or $-O^-$ M, wherein M is a metal ion or an ammonium ion, and (C) water;

(ii) polymerizing the organosiloxane of the emulsified mixture in the presence of an acid catalyst to produce a polyorganosiloxane having a weight-average molecular weight of at least 2 times the weight-average molecular weight of the organosiloxane; and

(iii) neutralizing the acid catalyst.

In step (i) of the method of preparing the silicone emulsion, a mixture comprising components (A'), (B'), and (C) is emulsified.

Component (A') is at least one organosiloxane having the formula $HO(R^1{}_2SiO)_nH$, wherein each $R^1$ is independently hydrocarbyl or substituted hydrocarbyl, and n has a value such that the organosiloxane has a weight-average molecular weight of from 92 to 100,000, alternatively from 350 to 50,000, alternatively from 1,000 to 10,000, as determined by gel permeation chromatography employing a refractive index detector and polystyrene standards. Also, the hydrocarbyl and substituted hydrocarbyl groups represented by $R^1$ are as described and exemplified above for $R^3$ in the formula of the surfactant, component (B) of the silicone emulsion.

Component (A') typically contains not greater than 2.5 % (w/w), alternatively not greater than 1.5% (w/w), alternatively not greater than 1.0% (w/w), of cyclic organosiloxane tetramer. If necessary, the concentration of cyclic organosiloxane oligomers, including tetramer, in component (A') can be reduced using conventional methods of evaporation. For example, the organosiloxane can be heated under reduced pressure using a thin-film evaporator.

Examples of organosiloxanes include, but are not limited to, α,ω-dihydroxypolydimethylsiloxane, 1,3-dihydroxytetramethyldisiloxane, and 1,7-dihydroxy-octamethyltetrasiloxane.

Component (A') can be a single organosiloxane or a mixture comprising two or more different organosiloxanes, each as described and exemplified above. Also, methods of preparing hydroxy-terminated organosiloxanes, such as hydrolysis and condensation of organohalosilanes or equilibration of organocyclosiloxanes, are well known in the art.

Component (B') is at least one surfactant having the formula:

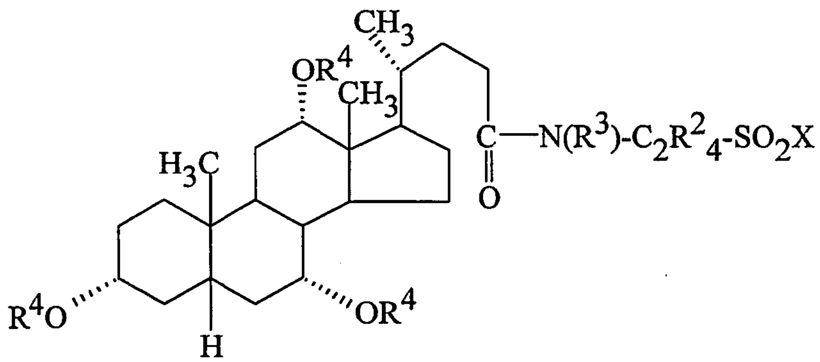

wherein each $R^2$ is independently -H or -F, $R^3$ is -H, hydrocarbyl, or substituted hydrocarbyl, each $R^4$ is independently $R^3$ or $-(CH_2CH_2O)_mR^3$, wherein m is from 1 to 20, and X is -OH or $-O^-$ M, wherein M is a metal ion or an ammonium ion. In the formula of component (B'), $R^2$, $R^3$, $R^4$ and the subscript m are as defined and exemplified above for component

(B) of the silicone emulsion.

Examples of surfactants suitable for use as component (B') include, but are not limited to, taurocholic acid, taurocholic acid sodium salt, taurocholic acid potassium salt, taurocholic acid lithium salt, taurocholic acid magnesium salt, and taurocholic acid triethanolamime salt.

Component (B') can be a single surfactant or mixture comprising two or more different surfactants, each as described above. For example, component (B') can be a single acid (i.e., X is -OH), a mixture of two or more different acids, a single salt (i.e., X is $-O^{-}$ M), a mixture of two or more different salts, or a mixture of at least one acid and at least one salt.

Methods of preparing cholic acid derivatives, including ethers and amides, suitable for use as component (B') are well known in the art.

The mixture comprising (A'), (B'), and (C) can further comprises at least one organosilane having at least one silicon-bonded hydrolysable group. As used herein the term "hydrolysable group" means the silicon-bonded group reacts with water in either the presence or absence of a catalyst at any temperature from room temperature (~23 ± 2 °C) to 100 °C within several minutes, for example thirty minutes, to form a silanol (Si-OH) group. Examples of hydrolysable groups represented include, but are not limited to, -Cl, -Br, $-OR^5$,$-OCH_2CH_2OR^5$, $CH_3C(=O)O-$, Et(Me)C=N-O-, $CH_3C(=O)N(CH_3)-$, and $-ONH_2$, wherein $R^5$ is $C_1$ to $C_8$ hydrocarbyl or $C_1$ to $C_8$ halogen-substituted hydrocarbyl.

The hydrocarbyl and halogen-substituted hydrocarbyl groups represented by $R^5$typically have from 1 to 8 carbon atoms, alternatively from 3 to 6 carbon atoms. Acyclic hydrocarbyl and halogen-substituted hydrocarbyl groups containing at least 3 carbon atoms can have a branched or unbranched structure. Examples of hydrocarbyl groups represented by $R^3$ include, but are not limited to, unbranched and branched alkyl, such as methyl, ethyl, propyl, 1-methylethyl, butyl, 1-methylpropyl, 2-methylpropyl, 1,1-dimethylethyl, pentyl, 1-methylbutyl, 1-ethylpropyl, 2-methylbutyl, 3-methylbutyl, 1,2-dimethylpropyl, 2,2-dimethylpropyl, hexyl, heptyl, and octyl; cycloalkyl, such as cyclopentyl, cyclohexyl, and methylcyclohexyl; phenyl; alkaryl, such as tolyl and xylyl; aralkyl, such as benzyl and phenethyl; alkenyl, such as vinyl, allyl, and propenyl; arylalkenyl, such as styryl; and alkynyl, such as ethynyl and propynyl. Examples of halogen-substituted hydrocarbyl groups represented by $R^5$ include, but are not limited to, 3,3,3-trifluoropropyl, 3- chloropropyl, chlorophenyl, and dichlorophenyl.

The groups in the organosilane other than the hydrolysable group(s) are typically hydrocarbyl or substituted hydrocarbyl groups, as described and exemplified above for the polyorganosiloxane, component (A), of the silicone emulsion.

Examples of organosilanes include, but are not limited to, methyltrimethoxysilane, methyltriethoxysilane, tetraethoxysilane, 3-aminopropylmethyldiethoxysilane, 3-aminopropyltriethoxysilane, N-(2-aminoethyl)-3-aminopropyltriethoxysilane, N-(2-aminoethyl)-3-aminopropyltrimethoxysilane, N-(2-aminoethyl)-3-aminopropyl methyldiethoxysilane, N-(2-aminoethyl)-3-aminopropylmethyldimethoxysilane, 3-chloropropyltriethoxysilane, 3-chloropropyltrimethoxysilane, 3-chloropropyl methyldiethoxysilane, 3-chloropropylmethyldimethoxysilane, 3-methacryloxypropyltriethoxysilane, 3-methacryloxypropyltrimethoxysilane, 3-acryloxypropylmethyldiethoxysilane, 3-acryloxypropylmethyldimethoxysilane, 3-glycidoxypropyltriethoxysilane, 3-glycidoxypropyltrimethoxysilane, 3-glycidoxypropylmethyldiethoxysilane, 3-glycidoxypropylmethyldimethoxysilane, 3-mercaptopropylmethyldiethoxysilane, 3-mercaptopropylmethyldimethoxysilane, 3-carboxypropylmethyldiethoxysilane, 3-carboxypropylmethyldimethoxysilane, p-vinylphenyltriethoxysilane, p-vinylphenyltrimethoxysilane, 2-(vinylphenyl)ethyltriethoxysilane, 2-(vinylphenyl)ethyltrimethoxysilane, 3-(p-isopropenylbenzoylamino)propyltriethoxysilane, 3-(p-isopropenylbenzoylamino)propyltrimethoxysilane, N-methacryloyl-N-methyl-3-aminopropyltriethoxysilane, N-methacryloyl-N-methyl-3-aminopropyltrimethoxysilane, N-lauroyl-N-methyl-3-aminopropylmethyldiethoxysilane, N-lauroyl-N-methyl-3-aminopropylmethyldimethoxysilane, N-acryloyl-N-methyl-3-aminopropyltriethoxysilane, N-acryloyl-N-methyl-3-aminopropyltrimethoxysilane, N-lauroyl-N-methyl-3-aminopropyltriethoxysilane, N-lauroyl-N-methyl-3-aminopropyltrimethoxysilane, N,N-bis (methacryloyl)-3-aminopropylmethyldiethoxysilane, N,N-bis (methacryloyl)-3-aminopropylmethyldimethoxysilane, N,N-bis(lauroyl)-3-aminopropyltriethoxysilane, and N,N-bis (lauroyl)-3-aminopropyltrimethoxysilane.

The mixture comprising (A'), (B'), and (C) can be emulsified using conventional equipment such as a homogenizer, colloidal mill, line mixer, sonolator, combination mixer, Turello mixer, or homogenizer-mixer. In this procedure, after coarse emulsification in an emulsifier, such as a homogenizer, colloidal mill, or line mixer, subsequent fine emulsification may be performed in a pressurized homogenizer or an ultrasonic homogenizer. If necessary, additional uniform emulsification and dispersion can then be conducted with the addition of water.

The mixture comprising (A'), (B), and (C) is typically emulsified at a temperature of from 5 to 75 °C, alternatively from 5 to 40 °C.

The emulsification time depends on many factors, including the structure of the organosiloxane, temperature, and type of equipment used to emulsify the mixture. The mixture is typically emulsified for a period of time sufficient to produce (siloxane) particles having a size of from 100 to 5,000 nm, alternatively from 200 to 3,000 nm, alternatively from 300 to 1,000 nm. For example, the mixture is typically emulsified for a period of from 1 to 60 min., alternatively from 1 to 30 min., alternatively from 1 to 10 min. As used here, particle size refers to the mean volume diameter defined by the

equation:

$$d_v = \left(\frac{\Sigma n_i d_i^3}{\Sigma n_i}\right)^{1/3}$$

where $d_v$ is the mean volume diameter of all the particle volumes forming the entire population, and ni is the number of particles in group i having midpoint diameter $d_i$.

The concentration of component (A') in the mixture is typically from 5 to 90% (w/w), alternatively from 10 to 75% (w/w), alternatively from 30 to 60% (w/w), based on the total weight of the mixture.

The concentration of component (B') in the mixture is typically from 0.1 to 20% (w/w), alternatively from 0.5 to 10% (w/w), alternatively from 0.5 to 5% (w/w), based on the weight of component (A').

The concentration of water in the mixture is typically from 30 to 1000% (w/w), alternatively from 40 to 400% (w/w), alternatively from 50 to 250% (w/w), based on the weight of component (A').

The concentration of the optional organosilane in the mixture is typically from 0 to 10% (w/w), alternatively from 0 to 5% (w/w), alternatively from 0 to 1% (w/w), based on the weight of component (A').

In step (ii) of the method of preparing the silicone emulsion, the organosiloxane of the emulsified mixture is polymerized in the presence of an acid catalyst to produce a polyorganosiloxane having a weight-average molecular weight of at least 2 times, alternatively at least 20 times, alternatively at least 200 times, the weight-average molecular weight of the organosiloxane.

The acid catalyst is the acid form of the surfactant, component (B'), in the emulsified mixture. The concentration of the acid catalyst is such that at least 10 mol% of the surfactant in the emulsified mixture is present as a sulfonic acid. When the surfactant in the emulsified mixture is present entirely as a salt or as a mixture containing a salt and less than 10 mol% of a sulfonic acid, an acid is typically added to the emulsified mixture in an amount sufficient to convert at least a portion of the salt to the corresponding sulfonic acid. Examples of acid catalysts include, but are not limited to, inorganic acids such as sulfuric acid, hydrochloric acid, phosphoric acid; and organic acids such as formic acid, acetic acid, and citric acid.

The polymerization reaction can be carried out in any standard vessel suitable for suspension polymerizations. The vessel is typically equipped with a means of agitation, such as stirring or mixing.

The polymerization is typically carried out at a temperature of from 5 to 75 °C, alternatively from 5 to 40 °C, alternatively from 5 to 25°C.

The polymerization time depends on several factors, including the structure of the organosiloxane, reaction temperature, and the desired molecular weight of the polyorganosiloxane. The polymerization is typically carried out for a period of time sufficient to produce a polyorganosiloxane having a weight-average molecular weight of at least 2 times, alternatively at least 20 times, alternatively at least 200 times, the weight-average molecular weight of the organosiloxane, as determined by gel permeation chromatograph employing a refractive index detector and polystyrene standards. For example, the polymerization time is typically from 2 to 72 h, alternatively from 6 to 48 hours, alternatively from 8 to 48 hours.

When the polymerization produces a polyorganosiloxane having the desired weight-average molecular weight, the acid catalyst is neutralized to terminate the polymerization reaction. The acid catalyst can be neutralized by adding a base to the polymerization reaction. Examples of bases include, but are not limited to, inorganic bases, such as sodium hydroxide, potassium hydroxide, ammonia, sodium carbonate, potassium carbonate, ammonium carbonate, and potassium acetate; and organic bases such as triethanolamine.

As stated above, the mixture comprising (A'), (B'), and (C) in step (i) of the method of preparing the silicone emulsion can further comprise at least one organosilane having at least one silicon-bonded hydrolysable group. Alternatively, the method of preparing the silicone emulsion can further comprise, after step (i) and before step (ii), treating the emulsified mixture with at least one organosilane having at least one silicon-bonded hydrolysable group, where the organosilane is as described and exemplified above.

The silicone emulsion of the invention can be used as an ingredient in cosmetic compositions, such as skin creams; foundation; eye shadow; body wash; and hair-care cosmetics such as shampoo, hair rinse, hair conditioner, hair treatment formulations, set lotions, blow styling aids, hair sprays, foam-type styling aids, gel-type styling aids, hair liquids, hair tonics, hair creams, hair growth aids, hair-nourishing aids, and hair dyes.

A cosmetic ingredient according to the present invention comprises the silicone emulsion and, optionally, one or more additive to improve its compounding stability in a cosmetic composition. Examples of additives include, but are not limited to, nonionic surfactants, anionic surfactants, pH-adjusting agents, antiseptics, mildew-proofing agents, and rust preventives.

Examples of nonionic surfactants include, but are not limited to, ethylene glycol fatty acid esters, polyethylene

glycol fatty acid esters, propylene glycol fatty acid esters, polypropylene glycol fatty acid esters, glycol fatty acid esters, trimethylolpropane fatty acid esters, pentaerythritol fatty acid esters, glucoside derivatives, glycerin alkyl ether fatty acid esters, trimethylolpropane oxyethylene alkyl ethers, fatty acid amides, alkylolamides, alkylamine oxides, lanolin and its derivatives, castor oil derivatives, hardened castor oil derivatives, sterol and its derivatives, polyoxyethylene alkyl ethers, polyoxyethylene alkyl allyl ethers, polyoxyethylene alkylamines, polyoxyethylene fatty acid amides, polyoxyethylene alkylolamides, polyoxyethylene diethanolamine fatty acid esters, polyoxyethylene trimethylolpropane fatty acid esters, polyoxyethylene alkyl ether fatty acid esters, polyoxyethylene polyoxypropylene glycols, polyoxyethylene polyoxypropylene alkyl ethers, polyoxyethylene polyoxypropylene polyhydric alcohol ethers, glycerin fatty acid esters, polyglycerin fatty acid esters, polyoxyethylene glycerin fatty acid esters, sorbitan fatty acid esters, polyoxyethylene sorbitan fatty acid esters, and sucrose fatty acid esters.

Examples of anionic surfactants include, but are not limited to, diethanolamine N-acyl-L-glutamate, triethanolamine N-acyl-L-glutamate, sodium N-acyl-L-glutamate, sodium alkanesulfonate, ammonium alkyl(12, 14, 16) sulfate, triethanolamine (1) alkyl(11, 13, 15)sulfate, triethanolamine (2) alkyl(11, 13, 15)sulfate, triethanolamine alkyl(12 14) sulfate, triethanolamine alkylsulfate solution, sodium alkyl(12, 13)sulfate, sodium alkylsulfate solution, sodium isethionate, sodium lactostearate, disodium undecylenoylamidoethyl sulfosuccinate, triethanolamine sulfooleate, sodium sulfooleate, disodium oleamido sulfosuccinate, potassium oleate, sodium oleate, morpholine oleate, oleyl sarcosine, oleylmethyl taurine sodium salt, potassium-containing soap base, potassium soap base solution, potassium soap, carboxylated polyoxyethylene tridodecyl ether, carboxylated polyoxyethylene; tridodecyl ether sodium salt (3 E.O.), triethanolamine N-(hardened tallow fatty acid)-acyl-L-glutamate, sodium N-(hardened tallow fatty acid)-acyl-L-glutamate, sodium (hardened coconut oil fatty acid) glyceryl sulfate, sodium diundecylenoylamidoethyl sulfosuccinate, sodium sulfostearate, potassium stearate, triethanolamine stearate, sodium stearate, sodium N-stearoyl-L- glutamate, disodium stearoyl-L-glutamate, stearoylmethyl taurine sodium salt, dioctyl sodium sulfosuccinate, dioctyl sodium sulfosuccinate solution, disodium polyoxyethylene monooleylamido sulfosuccinate (2 E.O.) solution, disodium polyoxyethylene lauroyl ethanolamido sulfosuccinate (5 E.O.), disodium lauryl sulfosuccinate, diethanolamide cetylsulfate, sodium cetylsulfate, soap base, sodium cetostearyl sulfate, triethanolamine tridecyl sulfate, potassium palmitate, sodium palmitate, palmitoylmethyl taurine sodium salt, solution (30%) of sodium salt of castor oil fatty acid, ammonium polyoxyethylene alkyl ether sulfate (3 E.O.) solution, diethanolamine polyoxyethylene alkyl(12, 13); ether sulfate (3 E.O.) solution, triethanolamine polyoxyethylene alkyl ether sulfate (3 E. O.) solution, triethanolamine polyoxyethylene alkyl (11, 13, 15) ether sulfate (1 E.O.), triethanolamine polyoxyethylene alkyl (12, 13) ether sulfate (3 E.O.), sodium polyoxyethylene alkyl ether sulfate (3 E.O.) solution, sodium polyoxyethylene alkyl (11, 13, 15) ether sulfate (1 E.O.), sodium polyoxyethylene alkyl (11 15) ether sulfate (3 E.O.), sodium polyoxyethylene alkyl (12, 13) ether sulfate (3 E.O.), sodium polyoxyethylene alkyl (12 14) ether sulfate (3 E.O.), sodium polyoxyethylene alkyl (12 15) ether sulfate (3 E.O.), disodium polyoxyethylene alkyl (12 14) sulfosuccinate (7 E.O.), sodium polyoxyethylene undecyl ether sulfate, sodium polyoxyethylene octyl phenyl ether sulfate solution, ammonium polyoxyethylene oleyl ether sulfate, disodium lauryl polyoxyethylene sulfosuccinate, sodium polyoxyethylene nonyl phenyl ether sulfate, sodium polyoxyethylene pentadecyl ether sulfate, triethanolamine polyoxyethylene myristyl ether sulfate, sodium polyoxyethylene myristyl ether sulfate, sodium polyoxyethylene myristyl ether sulfate (3 E.O.), sodium polyoxyethylene lauryl ether acetate (16 E.O.) solution, ammonium polyoxyethylene lauryl ether sulfate (2 E.O.), triethanolamine polyoxyethylene lauryl ether sulfate, sodium polyoxyethylene lauryl ether sulfate, diethanolamine myristyl sulfate, sodium myristyl sulfate, potassium myristate, sodium N-myristoyl- L-glutamate, sodium myristoylmethylaminoacetate, myristoylmethyl-β-alanine sodium salt solution, myristoylmethyl taurine sodium salt, medicinal soap, magnesium-triethanolamine cocoalkyl sulfate, triethanolamine N-cocoate acyl-L- glutamate, sodium N-cocoate acyl-L-glutamate, sodium coconut oil fatty acid ethyl ester sulfonate, potassium cocoate, potassium cocoate solution, sodium N-cocoate-tallowate acyl-L-glutamate, sarcosine cocoate, sarcosine triethanolamine cocoate, sarcosine sodium coca ate, triethanolamine cocoate, triethanolamine cocoate solution, sodium cocoate, sodium methyl alanine cocoate, sodium methyl alanine cocoate solution, potassium methyl taurine cocoate, sodium methyl taurine cocoate, sodium laurylaminodipropionate, sodium laurylaminodipropionate solution (30%), sodium lauryl sulfoacetate, sodium laurylbenzenesulfonate, laurylsulfuric acid, ammonium laurylsulfate, potassium laurylsulfate, diethanolamine laurylsulfate, triethanolamine laurylsulfate, sodium laurylsulfate, magnesium laurylsulfate, monoethanolamine laurylsulfate, potassium laurate, triethanolamine laurate, triethanolamine laurate solution, sodium laurate, triethanolamine laurate myristate, triethanolamine lauroyl-L-glutamate, sodium N-lauroyl-L-glutamate, lauroyl sarcosine, lauroyl sarcosine potassium salt, lauroyl sarcosine triethanolamine salt solution, lauroyl sarcosine sodium salt, lauroylmethyl-β-alanine sodium salt solution, lauroylmethyl taurine sodium salt, and lauroylmethyl taurine sodium salt solution.

Examples of pH-adjusting agents include, but are not limited to, hydrochloric acid, sulfuric acid, phosphoric acid, diammonium hydrogenphosphate, disodium hydrogenphosphate, dipotassium hydrogenphosphate, ammonium dihydrogenphosphate, sodium dihydrogenphosphate, potassium dihydrogenphosphate, trisodium phosphate, tripotassium phosphate, acetic acid, ammonium acetate, sodium acetate, potassium acetate, citric acid, sodium citrate, diammonium citrate, sodium carbonate, potassium carbonate, ammonium carbonate, sodium hydrogencarbonate, ammonium hydrogencarbonate, sodium hydroxide, potassium hydroxide, ammonia, and triethanolamine.

Examples of antiseptics, mildew-proofing agents, and rust preventives include, but are not limited to, benzoic acid, aluminum benzoate, sodium benzoate, isopropylmethylphenol, ethylhexanediol, lysozyme chloride, chlorhexidine hydrochloride, octylphenoxyethanol, orthophenylphenol, sodium perborate, photosensitive material No. 101, photosensitive material No. 201, photosensitive material No. 301, photosensitive material No. 401, chlorhexidine gluconate solution, cresol, chloramine T. chlorxylenol, chlorcresol, chlorphenesin, chlrohexidine, chlorobutanol, resorcin acetate, salicylic acid, sodium salicylate, domiphen bromide, zinc pyrithion, zinc pyrithion solution, sorbic acid, potassium sorbate, thianthol, thioxolone, thimol, chiram, dehydroacetic acid, sodium dehydroacetate, trichlorocarbanilide, trichlorohydroxydiphenyl ether, isobutyl paraoxybenzoate, isopropyl paraoxybenzoate, ethyl paraoxybenzoate, butyl paraoxybenzoate, propyl paraoxybenzoate, benzyl paraoxybenzoate, methyl paraoxybenzoate, sodium methyl paraoxybenzoate, parachlorphenol, sodium paraphenolsulfonate (dihydrate), halocarban, phenoxyethanol, phenol, hexachlorophane, mononitroguaiacol, mononitroguaiacol sodium, paradimethylaminostyrylheptylmethyllyazolinium iodide, lauryltrimethylammonium trichlorophenoxide, oxyquinoline sulfate, oxyquinoline phosphate, and resorcin.

Examples of other additives include, but are not limited to, avocado oil, almond oil, olive oil, cacao; butter, sesame oil, wheat germ oil, safflower oil, shea butter, turtle oil, tuna oil, persic oil, sunflower oil, grapeseed oil, macadamia nut oil, mink oil, egg yolk oil, Japan tallow, coconut oil, rosehip oil, hardened oil and other oils and fats; orange roughy oil, carnauba wax, candelilla wax, spermaceti wax, jojoba oil, montan wax, beeswax, lanolin and other waxes; liquid paraffin, Vaseline, paraffin, ceresin, microcrystalline wax, squalane, and other hydrocarbons; lauric acid, myristic acid, palmitic acid, stearic acid, oleic acid, behenic acid, undecylenic acid, oxystearic acid, linoleic acid, lanolic acid, synthetic fatty acids, and other higher fatty acids; ethyl alcohol, isopropyl alcohol, lauryl alcohol, cetyl alcohol, cetostearyl alcohol, stearyl alcohol, oleyl alcohol, behenyl alcohol, lanolin alcohol, hydrogenated lanolin alcohol, hexyldecanol, octyldodecanol, isostearyl alcohol, and other alcohols; cholesterol, dihydrocholesterol, phytosterol, and other sterols; ethyl linoleate, isopropyl myristate, lanolin fatty acid isopropyl, hexyl laurate, myristyl myristate, cetyl myristate, octyldodecyl myristate, decyl oleate, octyldodecyl oleate, hexyldecyl dimethyloctanoate, cetyl isooctanoate, cetyl palmitate, glycerin trimyristate, glycerin tri(capryl-caprate), propylene glycol dioleate, glycerin triisostearate, glycerin triisooctanoate, cetyl lactate, myristyl lactate, diisostearyl malate, and other fatty acid esters; glycerin, propylene glycol, 1,3-butylene glycol, polyethylene glycol, sodium d,1-pyrrolidonecarboxylate, sodium lactate, sorbitol, sodium hyaluronate, and other humectants; cationic surface active agents; betain- type, amino acid-type, imidazoline-type, lecithin and other amphoteric surface active agents; iron oxides and other colored pigments, zinc oxide, titanium oxide, zirconium oxide, and other white pigments; mica, talc, sericite, and other skin-color pigments; dimethylpolysiloxane, methylphenylpolysiloxane, octamethylcyclotetrasiloxane, decamethylcyclopentasiloxane, polyether- modified silicone oil, amino-modified silicone oil, and other silicone oils; demineralized water; carrageenan, alginic acid, I gum arable, traganth, pectin, starch, xanthan gum, polyvinyl alcohol, polyvinyl pyrrolidone, sodium polyacrylate, polyethylene glycol, and other thickeners, silicone-saccharides, silicone-acrylic copolymer, silicone resin, and acrylic polymers and other film-forming agents, and, furthermore, UV absorbers, anti-microbial agents, anti-inflammatory agents, anti-perspirant agents, fragrance, anti-oxidants, and propellants.

When the cosmetic ingredient of the invention is used in hair-care cosmetics, the cosmetic ingredient can comprise, in addition to the aforementioned additives, specialized additives, such as film forming agents, anti-freezing agents, oily components, emulsifiers, wetting agents, anti-dandruff agents, anti-oxidants, chelating agents, UV absorbers, fragrances, and colorants.

Examples of film-forming agents include, but are not limited to, polymers of (meth)acrylic radical-polymerizable monomers and their copolymers with silicone compounds, poly(N- acylalkyleneimine), poly(N-methylpyrrolidone), silicone resins modified by fluorine-containing organic groups or amino groups, non-functional silicone resins.

Examples of anti-freezing agents include, but are not limited to, ethanol, isopropyl alcohol, 1, 3-butylene glycol, ethylene glycol, propylene glycol, and glycerin.

Examples of oily components include, but are not limited to, microcrystalline wax, paraffin wax, spermaceti wax, beeswax, Japan wax, sugar cane wax, and other waxes or their mixtures, liquid paraffin, cc-olefin oligomers, squalane, squalene, and other hydrocarbon oils or their mixtures, cetanol, stearyl alcohol, isostearyl alcohol, hardened castor oil-derived alcohol, behenyl alcohol, lanolin alcohol, and other linear or branched saturated or unsaturated unsubstituted or hydroxy-substituted higher alcohols or their mixtures, palmitic acid, myristic acid, oleic acid, stearic acid, hydroxystearic acid, isostearic acid, behenic acid, castor oil fatty acid, coconut oil fatty acid, tallow fatty acid, and other linear or branched saturated or unsaturated unsubstituted or hydroxy-substituted higher fatty acids or their mixtures, olive oil, coconut oil, rape seed oil, palm oil, palm kernel oil, castor oil, hardened castor oil, peanut oil, beef tallow, hydrogenated beef tallow, jojoba oil, hardened jojoba oil, monostearic acid glyceride, monooleic acid glyceride, dipalmitic acid glyceride, 5 trimyristic acid glyceride, monooleic acid glyceride, oleyl oleate, isostearyl isostearate, palmityl behenate, isopropyl palmitate, stearyl acetate, dihydroxystearic acid ester, and other esters, linear, branched or cyclic low molecular silicone oils, amino- modified silicone oils, fatty acid-modified silicone oils, alcohol-modified silicone oils, polyether-modified silicone oils, phosphoric acid (phosphate)-containing silicone oils, fluorine-modified alkyl- containing silicone oils, alkyl-modified silicone oils, epoxy- modified silicone oils and other silicone oils, high molecular silicones, silicone

resins soluble in solvents, liquid or crude rubber-like at room temperature or possessing thermoplastic properties or their mixtures. The silicones are preferably latex-like, for example, one may suggest commonly used compounds, such as glycerin monostearate, sorbitan monopalmitate, polyoxyethylene cetyl ether, polyoxyethylene stearic acid ester and polyoxyethylene sorbitan laurate.

Examples of wetting agents include, but are not limited to, hexylene glycol, polyethylene glycol 600, sodium pyroglutamate, and glycerin.

Examples of anti-dandruff agents include, but are not limited to, sulfur, selenium sulfate, zinc pyridium-1-thiol-N-oxide, salicylic acid, 2,4,4'-trichloro-2'-hydroxydiphenyl ether, and 1-hydroxy-2-pyridone compounds.

Examples of anti-oxidants include, but are not limited to, BHA, BHT, and p-oryzanol.

Examples of chelating agents include, but are not limited to, ethylenediamine tetraacetate, citric acid, ethane-1-hydroxy-1,1-diphosphonic acid and their salts.

Examples of UV absorbers include, but are not limited to, benzophenone derivatives such as 2- hydroxy-4-methoxybenzophenone; benzotriazole derivatives such as 2- (2' 25 hydroxy-5'-methylphenyl)benzotriazole; and cinnamic acid ester.

Examples of other additives include, but are not limited to, glycerin, propylene glycol, dipropylene glycol, 1,3-butylene glycol, and other polyhydric alcohols; monoalkyltrimethylammonium salts; dialkyldimethylammonium salts and other quaternary ammonium salts, such as stearyltrimethylammonium chloride, behenyltrimethylammonium chloride, distearyldimethylammonium chloride, dibehenyldimethylammonium chloride; cationic surface active agents; amphoteric surfactants; squalane; lanolin; perfluoropolyether; cationic polymers and other tactile sensation improvers; propylene glycol; glycerin; sorbitol and other humectants; methylcellulose; carboxyvinyl polymer; hydroxyethylcellulose; polyoxyethylene glycol distearate; ethanol and other viscosity-adjusting agents; pearlescent agents; fragrances; pigments; dyes; propellants; vitamins; hair nourishing ingredients; hormones and other medicinal agents; trichlosan; trichlorocarban and other antimicrobial agents; potassium glycyrrhizinate; tocopherol acetate and other anti-inflammatory agents; zinc pirithion; octopyrox and other anti-dandruff agents; methylparaben; butylparaben and other antiseptics; propellants; and other components listed in the Encyclopedia of Shampoo Ingredients (Micelle Press, 1985).

## EXAMPLES

The following examples are presented to better illustrate the silicone emulsion and method of the present invention, but are not to be considered as limiting the invention, which is delineated in the appended claims. Unless otherwise noted, all parts and percentages reported in the examples are by weight. Also, $D_4$ is an abbreviation for octamethylcyclotetrasiloxane. The following methods and materials were employed in the examples:

### Determination of Molecular Weight and %$D_4$

A neutralized aliquot (0.1 g) from the polymerization reaction was placed in a 1 oz. glass vial. Ethylene glycol (10 g, histological grade) and 10 g of toluene (99.8%, HPLC grade) were added to the vial and the contents were thoroughly mixed using a laboratory vortex mixer for 45-60 seconds. The mixture was then centrifuged using an International Equipment Company Model HN-S centrifuge at 2600 rpm for 20 minutes. The toluene phase was then collected for gel permeation chromatography.

The chromatographic equipment consisted of a Waters 515 pump, a Waters 717 autosampler and a Waters 2410 differential refractometer. The separation was made with two (300 mm x 7.5 mm) Polymer Laboratories PLgel 5 $\mu$m Mixed-C columns (molecular weight separation range of 200 to 2,000,000), preceded by a PLgel 5 $\mu$m guard column (50 mm x 7.5 mm). The analyses were performed using HPLC grade toluene flowing at 1.0 mL/min. as the eluent, and the columns and detector were both at 45 °C. The toluene extract was transferred to a glass autosampler vial without filtering. An injection volume of 50 $\mu$L was used and data was collected for 25 minutes. Data collection and analyses were performed using ThermoLabsystems Atlas chromatography software and Polymer Laboratories Cirrus GPC software. Number-average and weight-average molecular weights were determined relative to a calibration curve (3rd order) created using polystyrene standards covering the molecular weight range of 580 - 1,300,000. The reported value for %$D_4$, determined by peak integration, refers to the weight percent of octamethylcyclotetrasiloxane, based on the total weight of siloxanes in the toluene extract. In Tables 1-3, below, the abbreviations $M_n$, Mw, and PD refer to number-average molecular weight, weight-average molecular weight, and polydisperisty ($M_w/M_n$), respectively

### Particle Size Analysis

Emulsion particle size was measured using dynamic light scattering on a Particle Sizing Systems Nicomp 370 Submicron Particle Sizer equipped with CW380 Version 1.51a software. Samples were measured in Kimble 6 x 50 mm borosilicate disposable culture tubes. The sample was diluted until an intensity of 200-400 kHz was achieved. Sample

data was collected for 5 minutes. All particle size measurements are mean volume diameters derived from the intensity-weighted particle size distribution obtained by the instrument. Mean volume diameter is defined by the equation:

$$d_v = \left( \frac{\Sigma n_i d_i^3}{\Sigma n_i} \right)^{1/3}$$

where $d_v$ is the mean volume diameter of all the particle volumes forming the entire population, and ni is the number of particles in group i having midpoint diameter $d_i$.

Emulsification

Emulsions in the Examples were prepared using a Fisher Scientific 550 Sonic Dismembrator, equipped with a 0.5" tip on the sonic probe.

Comparative Example 1

A mixture (40 g) was prepared by combining a hydroxy-endblocked polydimethylsiloxane having a weight-average molecular weight of 44560 (20 g), a 20% aqueous solution of sodium dodecyl sulfate (SDS) containing 22.55 mmol of SDS per liter of the polydimethylsiloxane, and deionized water. The surfactant solution was added to the polydimethylsiloxane, followed by the deionized water.

The mixture was vortexed for 10 to 15 seconds to form a rough emulsion. The rough emulsion was then exposed to the sonic dismembrator for 60 seconds at power setting 5. The vial was then capped and cooled under running water. The preceding sonication and cooling procedure was repeated five additional times.

A second mixture (40 g) containing the silanol-endblocked polydimethylsiloxane, SDS, and water was prepared and sonicated as described above. The two batches were then combined to give an emulsified mixture having a mean particle size of 267 nm.

A sample (15 g) of the emulsion was placed in a 2 oz. glass vial equipped with a stir bar. The vial was placed on a submersible magnetic stir plate in a Brinkman MGW/Lauda Model RM 20 Temperature Controlled Water Bath and the contents were allowed to equilibrate at 5 °C.

After equilibration, 0.1M $H_2SO_4$ was added in an amount sufficient to convert one-half the number of moles of SDS to the corresponding acid. The capped and stirred sample was kept at 5 °C for 72 hours, during which time 0.5 mL aliquots were removed at 1, 2, 4, 6, 8, 24, 48,and 72 hours. Each aliquot was immediately neutralized with 0.05M NaOH and subjected to analysis by GPC. Using three additional samples of the emulsion (15 g each), the former procedure was repeated at temperatures of 25 °C, 45 °C, and 65 °C. The analytical results are shown in Table 1.

Table 1

| | 5 °C | | | | 25 °C | | | |
|---|---|---|---|---|---|---|---|---|
| Time, h | Mn | Mw | PD | %D4 | Mn | Mw | PD | %D4 |
| 0 | 2490 | 4560 | 1.8 | 1.34 | 2490 | 4560 | 1.8 | 1.34 |
| 1 | 3190 | 5850 | 1.8 | 1.82 | 3940 | 7670 | 1.9 | 1.34 |
| 2 | 3830 | 8110 | 2.1 | 2.1 | 4790 | 12600 | 2.6 | 2.21 |
| 4 | 4480 | 11200 | 2.5 | 2.08 | 6920 | 28300 | 4.1 | 3.04 |
| 6 | 5130 | 14600 | 2.8 | 2.33 | 12700 | 42700 | 3.4 | 3.96 |
| 8 | 18100 | 61800 | 3.4 | 3.64 | 16000 | 58400 | 3.7 | 4.62 |
| 24 | 33500 | 139000 | 4.1 | 5.19 | 64600 | 170000 | 2.6 | 6.44 |
| 48 | 51400 | 216000 | 4.2 | 5.88 | 96800 | 244000 | 2.5 | 6.84 |
| 72 | 85800 | 276000 | 3.2 | 7.38 | 104000 | 278000 | 2.7 | 7.03 |

(continued)

| | 45°C | | | | 65 °C | | | |
|---|---|---|---|---|---|---|---|---|
| Time, h | Mn | Mw | PD | %D4 | Mn | Mw | PD | %D4 |
| 0 | 2490 | 4560 | 1.8 | 1.34 | 2490 | 4560 | 1.8 | 1.34 |
| 1 | 5600 | 13500 | 2.4 | 2.52 | 12900 | 30000 | 2.3 | 4.36 |
| 2 | 10200 | 27600 | 2.7 | 3.88 | 26700 | 50800 | 1.9 | 5.93 |
| 4 | 17900 | 54000 | 3.0 | 5.29 | 41500 | 75500 | 1.8 | 7.06 |
| 6 | 24200 | 77200 | 3.2 | 6.16 | 48100 | 90000 | 1.9 | 7.24 |
| 8 | 57700 | 106000 | 1.8 | 6.72 | 51800 | 97500 | 1.9 | 7.14 |
| 24 | 86600 | 165000 | 1.9 | 7.19 | 81000 | 149000 | 1.8 | 7.2 |
| 48 | 88100 | 177000 | 2.0 | 7.27 | 77900 | 141000 | 1.8 | 7.11 |
| 72 | 96500 | 184000 | 1.9 | 7.17 | 59900 | 108000 | 1.8 | 7.33 |

Comparative Example 2

A silicone emulsion was prepared at a temperature of 25 °C according to the method of Comparative Example 1, except the SDS was replaced with sodium methyl cocoyl taurate, and the hydroxyl-endblocked polydimethylsiloxane having a weight-average molecular weight of 4560 was replaced with a hydroxy-endblocked polydimethylsiloxane having a weight-average molecular weight of 5060. The emulsified mixture had a mean particle size of 301 nm. The analytical results are shown in Table 2.

Table 2

| | 25°C | | | |
|---|---|---|---|---|
| Time, h | Mn | Mw | PD | %D4 |
| 0 | 2580 | 5060 | 2.0 | 1.1 |
| 1 | 3470 | 6030 | 1.7 | 1.0 |
| 2 | 4260 | 7870 | 1.8 | 1.4 |
| 4 | 5780 | 13500 | 2.3 | 1.7 |
| 6 | 7280 | 20200 | 2.8 | 2.3 |
| 8 | 8980 | 28600 | 3.2 | 2.6 |
| 24 | 33000 | 106000 | 3.2 | 4.6 |
| 48 | 77600 | 200000 | 2.6 | 6.0 |
| 96 | 95300 | 261000 | 2.7 | 6.6 |

Example 1

A silicone emulsion was prepared according to the method of Comparative Example 1, except the SDS was replaced with sodium taurocholate (95%), and the hydroxyl-endblocked polydimethylsiloxane having a weight-average molecular weight of 4560 was replaced with a hydroxy-endblocked polydimethylsiloxane having a weight-average molecular weight of 5060. The emulsified mixture had a mean particle size of 273 nm. The analytical results are shown in Table 3.

Table 3

| | 5 °C | | | | 25 °C | | | |
|---|---|---|---|---|---|---|---|---|
| Time, h | Mn | Mw | PD | %D4 | Mn | Mw | PD | %D4 |
| 0 | 2580 | 5060 | 2.0 | 1.12 | 2580 | 5060 | 2.0 | 1.12 |
| 1 | 2810 | 5140 | 1.8 | 2.02 | 3170 | 5800 | 1.8 | 1.4 |
| 2 | 3140 | 5940 | 1.9 | 1.64 | 3670 | 7280 | 2.0 | 1.53 |
| 4 | 3440 | 6860 | 2.0 | 1.77 | 4780 | 12400 | 2.6 | 1.64 |
| 6 | 3750 | 8580 | 2.3 | 1.8 | 5890 | 19400 | 3.3 | 1.88 |
| 8 | 6010 | 25100 | 4.2 | 2.12 | 6670 | 27000 | 4.0 | 1.97 |
| 24 | 13000 | 65200 | 5.0 | 2.67 | 20900 | 87500 | 4.2 | 2.9 |
| 48 | 30400 | 119000 | 3.9 | 2.59 | 60900 | 166000 | 2.7 | 3.66 |
| 72 | 64300 | 196500 | 3.1 | 3.66 | 82800 | 213000 | 2.6 | 4.4 |
| | 45 °C | | | | 65 °C | | | |
| Time, h | Mn | Mw | PD | %D4 | Mn | Mw | PD | %D4 |
| 0 | 2580 | 5060 | 2.0 | 1.12 | 2580 | 5060 | 2.0 | 1.12 |
| 1 | 4010 | 7670 | 1.9 | 1.22 | 5870 | 13300 | 2.3 | 1.52 |
| 2 | 4980 | 11800 | 2.4 | 1.93 | 7180 | 21800 | 3.0 | 2.76 |
| 4 | 7210 | 23700 | 3.3 | 2.52 | 15200 | 40800 | 2.7 | 3.7 |
| 6 | 12100 | 37800 | 3.1 | 2.77 | 29100 | 56500 | 1.9 | 4.03 |
| 8 | 40400 | 69700 | 1.7 | 3.03 | 35400 | 70000 | 2.0 | 4.51 |
| 24 | 64600 | 122000 | 1.9 | 4.82 | 52700 | 98900 | 1.9 | 5.57 |
| 48 | 79500 | 156000 | 2.0 | 5.88 | 52800 | 105000 | 2.0 | 5.84 |
| 72 | 87000 | 166000 | 1.9 | 6.25 | 51400 | 104000 | 2.0 | 6.13 |

Figure 1 shows a plot of %$D_4$ versus weight-average molecular weight for the silicone emulsions of Comparative Example 1, Comparative Example 2, and Example 1 prepared at 25 °C. In Figure 1, the abbreviations SDS, SMCT, and STC represent sodium dodecyl sulfate, sodium methyl cocoyl taurate, and sodium taurocholate, respectively.

## Claims

**1.** A silicone emulsion, comprising:

(A) a polyorganosiloxane containing not greater than 10% (w/w) of cyclic organosiloxane tetramer;
(B) a surfactant having the formula:

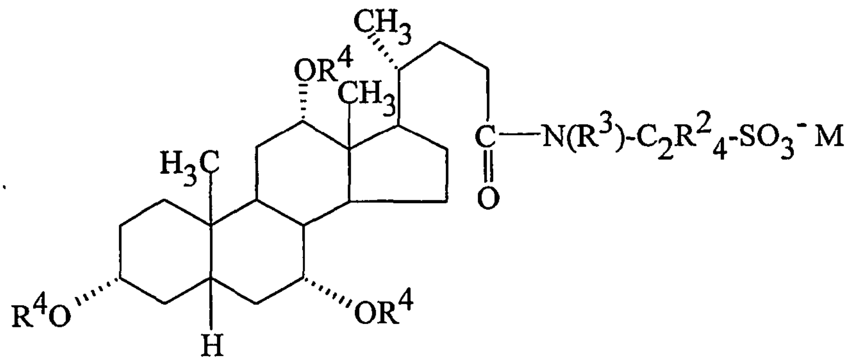

wherein each $R^2$ is independently -H or -F, $R^3$ is -H, hydrocarbyl, or substituted hydrocarbyl, each $R^4$ is independently $R^3$ or $-(CH_2CH_2O)_mR^3$, wherein m is from 1 to 20, and M is a metal ion or an ammonium ion; and (C) water.

2. The silicone emulsion according to claim 1, wherein the polyorganosiloxane of component (A) has a weight-average molecular weight of from 50,000 to 5,000,000.

3. The silicone emulsion according to claim 1, wherein component (A) contains not greater than 3.5% (w/w) of cyclic organosiloxane tetramer.

4. The silicone emulsion according to claim 1, wherein component (B) is a salt of taurocholic acid.

5. The silicone emulsion according to claim 1, wherein the concentration of component (B) is from 0.1 to 50 parts by weight per 100 parts by weight of component (A).

6. A cosmetic ingredient comprising the silicone emulsion according to claim 1.

7. The cosmetic ingredient according to claim 6, further comprising an additive selected from nonionic surfactants, anionic surfactants, pH-adjusting agents, antiseptics, mildew-proofing agents, and rust preventives.

8. The cosmetic ingredient according to claim 6, further comprising an additive selected from film forming agents, antifreezing agents, oily components, emulsifiers, wetting agents, anti-dandruff agents, anti-oxidants, chelating agents, UV absorbers, fragrances, and colorants.

9. A method of preparing a silicone emulsion, the method comprising:

(i) emulsifying a mixture comprising (A') an organosiloxane having the formula $HO(R^1{}_2SiO)_nH$, where each $R^1$ is independently hydrocarbyl or substituted hydrocarbyl, and n has a value such that the organosiloxane has a weight-average molecular weight of from 92 to 100,000; (B') a surfactant having the formula:

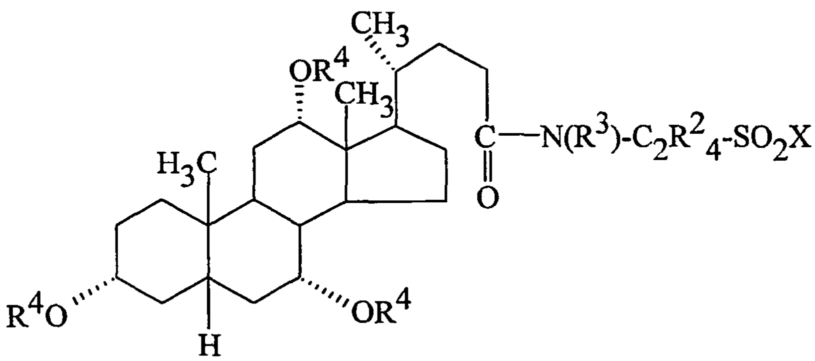

wherein each $R^2$ is independently -H or -F, $R^3$ is -H, hydrocarbyl, or substituted hydrocarbyl, each $R^4$ is independently $R^3$ or $-(CH_2CH_2O)_mR^3$, wherein m is from 1 to 20, and X is -OH or $-O^-$ M, wherein M is a metal ion or an ammonium ion, and (C) water;
(ii) polymerizing the organosiloxane of the emulsified mixture in the presence of an acid catalyst to produce a polyorganosiloxane having a weight-average molecular weight of at least 2 times the weight-average molecular weight of the organosiloxane; and
(iii) neutralizing the acid catalyst.

**10.** The method according to claim 9, wherein component (B') is selected from taurocholic acid, a salt of taurocholic acid, or a mixture thereof.

**11.** The method according to claim 9, wherein the mixture of step (i) further comprises at least one organosilane having at least one silicon-bonded hydrolysable group.

**12.** The method according to claim 9, further comprising, after step (i) and before step (ii), treating the emulsified mixture with at least one organosilane having at least one silicon-bonded hydrolysable

**13.** The method according to claim 9, wherein the mixture of step (ii) is emulsified for a period of time sufficient to produce a particle size of from 200 to 3,000 nm.

**14.** The method according to claim 9, wherein the polyorganosiloxane has a weight-average molecular weight of at least 20 times the weight-average molecular weight of the organosiloxane.

**15.** A silicone emulsion prepared according to the method of claim 9.

**Patentansprüche**

**1.** Eine Siliconemulsion, enthaltend:

(A) ein Polyorganosiloxan, enthaltend nicht mehr als 10% (w/w) eines cyclischen Organosiloxantetramers;
(B) ein oberflächenaktives Mittel mit der Formel:

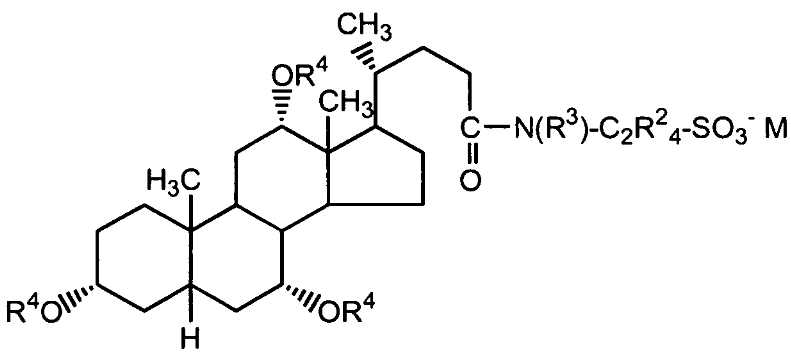

worin jedes $R^2$ unabhängig voneinander -H oder -F ist, $R^3$ gleich -H, Hydrocarbyl oder substituiertes Hydrocarbyl ist, jedes $R^4$ unabhängig voneinander $R^3$ oder-$(CH_2CH_2O)_mR^3$ ist, worin m von 1 bis 20 ist und M ein Metallion oder ein Ammoniumion ist; und
(C) Wasser.

**2.** Die Siliconemulsion gemäß Anspruch 1, wobei das Polyorganosiloxan der Komponente (A) ein gewichtsmittleres Molekulargewicht von 50.000 bis 5.000.000 hat.

**3.** Die Siliconemulsion gemäß Anspruch 1, wobei die Komponente (A) nicht mehr als 3,5% (w/w) an cyclischem Organosiloxantetramer enthält.

4. Die Siliconemulsion gemäß Anspruch 1, wobei die Komponente (B) ein Salz der Taurocholinsäure ist.

5. Die Siliconemulsion gemäß Anspruch 1, wobei die Konzentration der Komponente (B) von 0,1 bis 50 Gewichtsteile pro 100 Gewichtsteile der Komponente (A) ist.

6. Ein kosmetischer Bestandteil, enthaltend die Siliconemulsion gemäß Anspruch 1.

7. Der kosmetische Bestandteil gemäß Anspruch 6, außerdem enthaltend ein Additiv, ausgewählt aus nichtionischen oberflächenaktiven Mitteln, anionischen oberflächenaktiven Mitteln, Mitteln zum Einstellen des pH, Antiseptika, Mildew-Proofing Agents WEISS ICH NICHT MUSS MAN NACHGUCKEN; und RUST WEISS ICH AUCH NICHT? vorbeugenden Mitteln.

8. Der kosmetische Bestandteil gemäß Anspruch 6, außerdem enthaltend ein Additiv, ausgewählt aus filmbildenden Mitteln, Frostschutzmitteln, Ölkomponenten, Emulgatoren, Befeuchtungsmitteln, ANTI DANDRUFF WEISS ICH NICHT Mitteln, Antioxidantien, Chelatoren, UV-Absorbern, Duftstoffen und Färbemitteln.

9. Ein Verfahren zum Herstellen einer Siliconemulsion, wobei das Verfahren umfasst:

(i) Emulgieren einer Mischung, enthaltend (A') ein Organosiloxan mit der Formel $HO(R^1{}_2SiO)_nH$, wobei jedes $R^1$ unabhängig voneinander Hydrocarbyl oder substituiertes Hydrocarbyl ist und n einen solchen Wert hat, dass das Organosiloxan ein gewichtsmittleres Molekulargewicht von 92 bis 100.000 hat; (B') ein oberflächenaktives Mittel mit der Formel

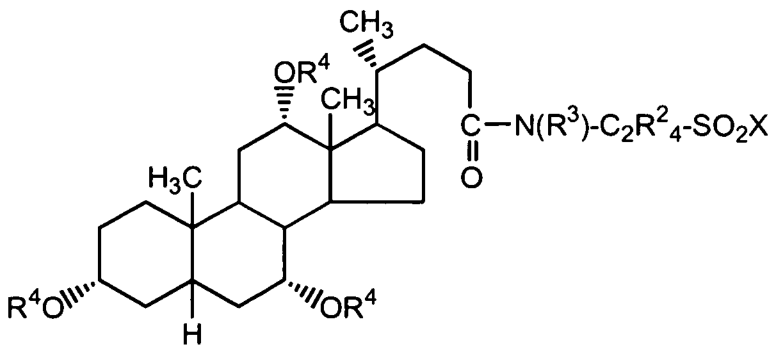

worin jedes $R^2$ unabhängig voneinander-H oder -F ist, $R^3$ gleich -H, Hydrocarbyl oder substituiertes Hydrocarbyl ist, jedes $R^4$ unabhängig voneinander $R^3$ oder $-(CH_2CH_2O)_mR^3$ ist, worin m von 1 bis 20 ist, und X gleich -OH oder $-O^-$ M ist, wobei M ein Metallion oder ein Ammoniumion ist, und (C) Wasser;

(ii) Polymerisieren des Organosiloxans der emulgierten Mischung in Gegenwart eines sauren Katalysators, um ein Polyorganosiloxan mit einem gewichtsmittleren Molekulargewicht von wenigstens dem Zweifachen des gewichtsmittleren Molekulargewichts des Organosiloxans herzustellen; und

(iii) Neutralisieren des sauren Katalysators.

10. Das Verfahren gemäß Anspruch 9, wobei die Komponente (B') ausgewählt ist aus Taurocholinsäure, einem Salz der Taurocholinsäure oder einer Mischung daraus.

11. Das Verfahren gemäß Anspruch 9, wobei die Mischung aus Schritt (i) außerdem wenigstens ein Organosilan mit wenigstens einer siliciumgebundenen hydrolysierbaren Gruppe enthält.

12. Das Verfahren gemäß Anspruch 9, außerdem umfassend nach Schritt (i) und vor Schritt (ii) das Behandeln der emulgierten Mischung mit wenigstens einem Organosilan mit wenigstens einer siliciumgebundenen hydrolysierbaren Gruppe. GRUPPE FEHLT IM A.12

13. Das Verfahren gemäß Anspruch 9, wobei die Mischung aus Schritt (ii) für eine Zeitdauer emulgiert wird, die ausreicht,

um eine Partikelgröße von 200 bis 3.000 nm herzustellen.

**14.** Das Verfahren gemäß Anspruch 9, wobei das Polyorganosiloxan ein gewichtsmittleres Molekulargewicht von wenigstens dem Zwanzigfachen des gewichtsmittleren Molekulargewichts des Organosiloxans hat.

**15.** Eine Siliconemulsion, hergestellt gemäß dem Verfahren nach Anspruch 9.

**Revendications**

**1.** Emulsion de silicone, comprenant :

(A) un polyorganosiloxane ne contenant pas plus de % (p/p) de tétramère d'organosiloxane cyclique ;
(B) un tensioactif de formule :

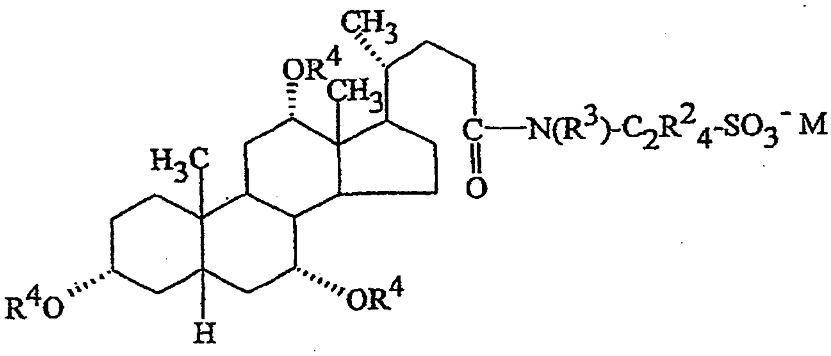

dans laquelle chaque $R^2$ est indépendamment -H ou -F, $R^3$ est -H, un groupe hydrocarbyle ou un groupe hydrocarbyle substitué, chaque $R^4$ est indépendamment $R^3$ ou $-(CH_2CH_2O)_mR^3$, dans laquelle m vaut 1 à 20 et M est un ion métallique ou un ion ammonium ; et
(C) de l'eau.

**2.** Emulsion de silicone selon la revendication 1, dans laquelle le polyorganosiloxane du composant (A) a une masse moléculaire moyenne en poids de 50 000 à 5 000 000.

**3.** Emulsion de silicone selon la revendication 1, dans laquelle le composant (A) ne contient pas plus de 3,5 % (p/p) de tétramère d'organosiloxane cyclique.

**4.** Emulsion de silicone selon la revendication 1, dans laquelle le composant (B) est un sel d'acide taurocholique.

**5.** Emulsion de silicone selon la revendication 1, dans laquelle la concentration du composant (B) est de 0,1 à 50 parties en poids pour 100 parties en poids du composant (A).

**6.** Ingrédient cosmétique comprenant l'émulsion de silicone selon la revendication 1.

**7.** Ingrédient cosmétique selon la revendication 6, comprenant en outre un additif choisi parmi les tensioactifs non ioniques, les tensioactifs anioniques, les agents régulateurs de pH, les antiseptiques, les agents antimoisissures et les protecteurs antirouille.

**8.** Ingrédient cosmétique selon la revendication 6, comprenant en outre un additif choisi parmi des agents filmogènes, des agents antigel, des composants huileux, des émulsifiants, des agents mouillants, des agents antipelliculaires, des antioxydants, des agents chélateurs, des absorbeurs d'UV, des parfums et des colorants.

**9.** Procédé pour préparer une émulsion de silicone, le procédé comprenant :

(i) la mise en émulsion d'un mélange comprenant (A') un organosiloxane de formule HO $(R^1{}_2SiO)_nH$, où chaque

$R^1$ est indépendamment un groupe hydrocarbyle ou un groupe hydrocarbyle substitué et n a une valeur telle que l'organosiloxane a une masse moléculaire moyenne en poids de 92 à 100 000 ; (B') un tensioactif de formule :

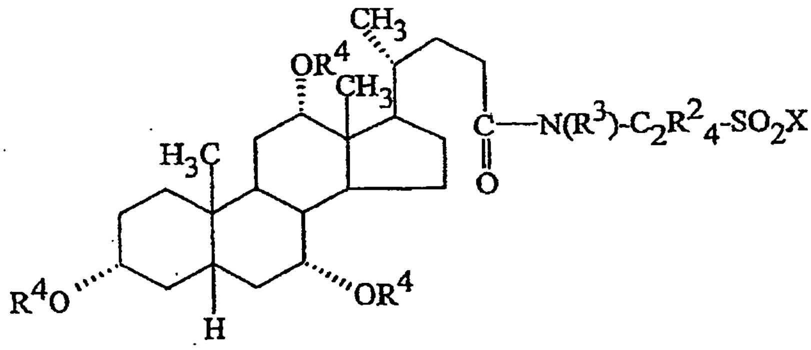

dans laquelle chaque $R^2$ est indépendamment -H ou -F, $R^3$ est -H, un groupe hydrocarbyle ou un groupe hydrocarbyle substitué, chaque $R^4$ est indépendamment $R^3$ ou - $(CH_2CH_2O)_mR^3$, dans laquelle m vaut 1 à 20, et X est -OH ou -$O^-M$, dans laquelle M est un ion métallique ou un ion ammonium, et (C) de l'eau ;
(ii) la polymérisation de l'organosiloxane du mélange émulsifié en présence d'un catalyseur acide pour produire un polyorganosiloxane ayant une masse moléculaire moyenne en poids représentant au moins le double de la masse moléculaire moyenne en poids de l'organosiloxane ; et
(iii) la neutralisation du catalyseur acide.

**10.** Procédé selon la revendication 9, dans lequel le composant (B') est choisi parmi l'acide taurocholique, un sel de l'acide taurocholique ou un de ses mélanges.

**11.** Procédé selon la revendication 9, dans lequel le mélange de l'étape (i) comprend en outre au moins un organosilane ayant au moins un groupe hydrolysable lié à un atome de silicium.

**12.** Procédé selon la revendication 9, comprenant en outre, après l'étape (i) et avant l'étape (ii), le traitement du mélange émulsifié avec au moins un organosilane ayant au moins un groupe hydrolysable lié à un atome de silicium.

**13.** Procédé selon la revendication 9, dans lequel le mélange de l'étape (ii) est émulsifié pendant une période suffisante pour produire une granulométrie de 200 à 3 000 nm.

**14.** Procédé selon la revendication 9, dans lequel le polyorganosiloxane a une masse moléculaire moyenne en poids représentant au moins 20 fois la masse moléculaire moyenne en poids de l'organosiloxane.

**15.** Emulsion de silicone préparée selon le procédé de la revendication 9.

Figure 1

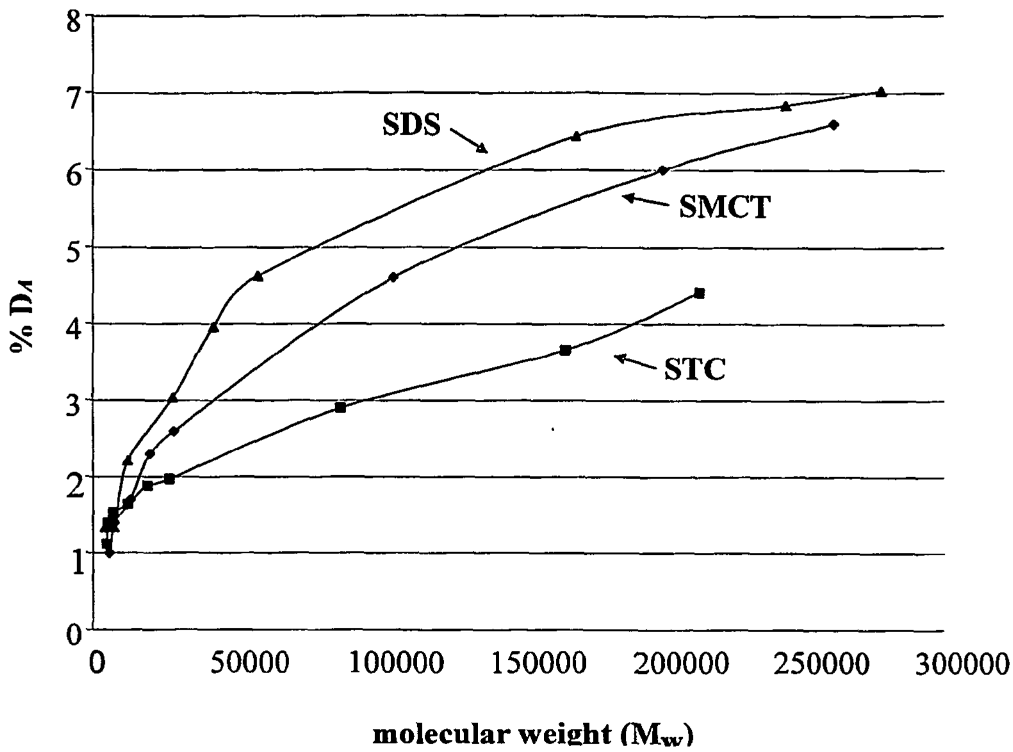
8
7
6
5
4
3
2
1
0
% DA
SDS
SMCT
STC
0
50000
100000
150000
200000
250000
300000
molecular weight (Mw)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 66273105 P
- US 662731 P


### Non-patent literature cited in the description


- Encyclopedia of Shampoo Ingredients. Micelle Press, 1985